# FASCICULE DE BREVET EUROPEEN

(11) **EP 1 697 740 B1**
(45) Date de publication et mention de la délivrance du brevet: **10.02.2010**
(21) Numéro de dépôt: 04816411.5
(22) Date de dépôt: 17.12.2004
(51) Int. Cl.: G01N 33/28

(54) **PROCÉDÉ; ET DISPOSITIF DE SUIVI DE LA DILUTION DE L'HUILE LUBRIFIANTE PAR LE CARBURANT DANS UN MOTEUR À COMBUSTION INTERNE**
VERFAHREN UND VORRICHTUNG ZUR ÜBERWACHUNG DER VERDÜNNUNG DES SCHMIERÖLS MIT DEM BRENNSTOFF IN EINEM VERBRENNUNGSMOTOR
METHOD AND DEVICE FOR MONITORING THE DILUTION OF THE LUBRICATING OIL BY THE FUEL IN AN INTERNAL COMBUSTION ENGINE

(30) Priorité: 23.12.2003 FR 0315260
(43) Date de publication de la demande: 06.09.2006
(73) Titulaire: TOTAL RAFFINAGE MARKETING, 92800 Puteaux (FR); Delta Services Industriels SPRL, 7503 Froyennes (BE)
(72) Inventeur: DEQUENNE, Bernard, F-69005 Lyon (FR)
(74) Mandataire: Doressamy, Clarisse
(86) Numéro de dépôt international: PCT/FR2004/003276
(87) Numéro de publication internationale: WO 2005/071403

(56) Documents cités:
- FR-A- 1 500 048
- GB-A- 1 095 056
- US-A- 2 957 986
- US-A- 4 048 497
- US-A- 4 321 056
- US-A- 5 445 964

## Description

La présente invention concerne un procédé de détermination du taux de dilution de l'huile de lubrification d'un moteur à combustion interne par le carburant, par mesure de la radioactivité d'un traceur radioactif introduit dans l'huile de lubrification ou dans le carburant, ainsi qu'un dispositif tel qu'un banc d'essai d'un moteur à combustion interne, permettant de mettre en oeuvre ce procédé.

On sait l'importance que présente aussi bien pour les fabricants d'automobiles que pour les producteurs d'huiles lubrifiantes et/ou d'additifs fonctionnels pour huiles moteurs la connaissance précise des phénomènes de dilution des lubrifiants par les carburants au sein des nouvelles générations de moteurs à injection directe, notamment ceux à allumage commandé ou à allumage par compression.

Ainsi, dans un moteur à allumage par compression, appelé communément moteur diesel, le principe d'alimentation en carburant par injection directe au sein de la chambre de combustion est à l'origine d'un transfert d'une partie du carburant vers le bas moteur où il se mélange à l'huile lubrifiante.

Ce transfert de carburant vers le système de lubrification du moteur est encore accentué pour les moteurs équipés de systèmes de post-traitement des gaz d'échappement, tels que des filtres à particules ou des pots catalytiques. En effet, dans les moteurs équipés de tels systèmes de post-traitement, des injections supplémentaires de carburant peuvent être réalisées au niveau des chambres de combustion à un moment où le carburant n'y sera pas brûlé mais envoyé vers la ligne d'échappement où il servira pour régénérer les systèmes de post-traitement des gaz d'échappement, par exemple pour la combustion des suies accumulées dans les filtres à particules ou pour modifier l'état d'oxydation du milieu à l'intérieur du système catalyseur. Le taux de dilution de l'huile lubrifiante par le carburant peut ainsi atteindre des valeurs de 10% en volume et plus.

L'introduction de carburant dans le circuit d'huile de lubrification a pour conséquence, d'une part, la dégradation des caractéristiques du lubrifiant, par exemple une réduction de sa viscosité, une dilution des additifs, et, d'autre part, une augmentation du volume présent au sein du carter d'huile. Il en résulte une altération du fonctionnement du moteur qui se manifeste par une pression d'huile réduits et une consommation d'huile anormalement élevée et aboutit, à terme, à une usure accrue des pièces mécaniques, voire même à la casse du moteur.

Or, l'utilisation de systèmes de post-traitement des gaz d'échappement tend à se généraliser en raison des normes anti-pollution de plus en plus sévères et la résolution du problème de dilution de l'huile de lubrification évoqué ci-dessus constitue par conséquent un défi important pour l'industrie automobile.

Les constructeurs automobiles doivent et devront donc réaliser de nombreux essais pour mettre au point des moteurs présentant une dilution contrôlée et si possible minimale de l'huile de lubrification par le carburant injecté au niveau des chambres de combustion.

Il existe un certain nombre de techniques telles que la chromatographie gazeuse sur colonne remplie ou capillaire permettant de doser la quantité de carburant dans des échantillons d'huile prélevés au sein d'un moteur sur un banc d'essai. Ces techniques d'analyse sont toutefois discontinues, relativement complexes, consomment l'échantillon d'huile analysé qui ne pourra plus être réintroduit dans le circuit de lubrification et demandent un temps d'analyse relativement long.

Le document US 2 957 986 divulgue un procédé et dispositif pour le suivi de la consommation d'huile d'un moteur à combustion interne par mesure de la radioactivité émise avec le gaz d'échappement. Le radioisotope utilisé est le ¹⁴C, sous forme d'hydrocarbures marqués. Le ¹⁴C se retrouve sous forme de ¹⁴CO₂ dans les gaz d'échappement et est piégé par précipitation sous forme de carbonate. Le carbonate accumulé sur une certaine période subit ensuite une décomposition thermique libérant du ¹⁴CO₂.

Le document GB 1 095 056 divulgue un procédé et un dispositif pour déterminer la consommation d'huile de carter d'un moteur par mesure de la radioactivité s'échappant par les gaz d'échappement du moteur. Ce procédé et dispositif utilisent un piège permettant d'accumuler le radiotraceur. Ce piège comprend une solution d'hydroxyde de sodium qui capte le radiotraceur (brome radioactif sous forme de HBr) par réaction chimique.

La Demanderesse a mis au point une méthode d'évaluation de la dilution de l'huile de lubrification par le carburant qui est relativement plus simple et plus rapide que les techniques connues, ne consomme pas l'échantillon analysé et qui peut fonctionner en continu, en cours de fonctionnement du moteur, de manière à fournir quasiment en temps réel le taux de dilution de l'huile de lubrification par le carburant.

Ce procédé d'analyse est basé sur la mesure de la radioactivité d'un échantillon d'huile, cette radioactivité étant introduite dans le système sous forme d'un traceur radioactif présent soit dans l'huile de lubrification dont il s'agit d'analyser la dilution, soit dans le diluant, à savoir le carburant alimentant le moteur. Dans certaines conditions, cette radioactivité reflête en effet parfaitement la quantité d'huile de lubrification ou la quantité de carburant dans l'échantillon d'huile analysé et permet par conséquent, grâce à un calcul simple réalisé par ordinateur, d'obtenir directement le taux de dilution de l'huile par le carburant.

La présente invention a donc pour objet un procédé de détermination du taux de dilution de l'huile de lubrification d'un moteur à combustion interne par le carburant, caractérisé par le fait que
- l'on marque avec un traceur radioactif soit l'huile de lubrification soit le carburant,
- que l'on mesure, de préférence en continu et en cours de fonctionnement du moteur, à l'aide d'un détecteur sensible au rayonnement radioactif émis par le traceur radioactif, la radioactivité d'un échantillon d'huile, et
- que l'on transmet les résultats de ces mesures vers un ordinateur qui calcule à partir de ces résultats le taux de dilution de l'huile de lubrification par le carburant.
   L'invention a également pour objet un dispositif permettant de mettre en oeuvre un tel procédé d'analyse, en particulier un banc d'essai d'un moteur à combustion interne, comprenant
- un moteur à combustion interne, lubrifié par une huile de lubrification et alimenté par un mélange air/carburant, soit l'huile de lubrification soit le carburant contenant un traceur radioactif,
- un moyen permettant le prélèvement temporaire et la réinjection, en continu ou discontinu, d'un échantillon d'huile du circuit d'huile du moteur,
- à proximité immédiate de ce moyen de prélèvement temporaire et de réinjection, un détecteur sensible au rayonnement radioactif émis par le traceur radioactif présent dans l'échantillon d'huile, et
- relié audit détecteur, un ordinateur programmé pour calculer, à partir des résultats des mesures de radioactivité de l'échantillon d'huile, fournis par ledit détecteur, le taux de dilution de l'huile de lubrification par le carburant.

Pour décrire plus en détail le procédé et le dispositif de la présente invention, on utilisera par la suite de manière parfaitement équivalente les termes « huile lubrifiante », « huile de lubrification » ou « lubrifiant » pour désigner l'huile non diluée ou la fraction d'huile dans le mélange huile/carburant qu'il s'agit d'analyser. Par contre, le terme « échantillon d'huile » est utilisé systématiquement pour désigner l'échantillon dérivé ou prélevé provisoirement dont on mesure la radioactivité. Au début de l'essai moteur, cet « échantillon d'huile » est bien entendu constitué exclusivement de lubrifiant et ne contient pas encore de carburant.

Il convient de noter en outre que le terme « huile de lubrification » ou un de ses équivalents, désigne le produit lubrifiant final, c'est-à-dire la base lubrifiante contenant l'ensemble des additifs fonctionnels éventuellement présents et éventuellement le traceur radioactif qui, comme on verra ci-après, peut être un de ces additifs fonctionnels.

Le procédé de détermination du taux de dilution de l'huile lubrifiante dans un moteur par le carburant ne peut pas être mis en oeuvre avec n'importe quel traceur radioactif. Celui-ci doit remplir notamment les conditions énoncées dans les paragraphes ci-après.

Le radiotraceur ne doit pas perturber le fonctionnement du moteur ou modifier de manière gênante les propriétés physico-chimiques de l'huile de lubrification ou du carburant. Pour cela il doit notamment soit être chimiquement inerte vis-à-vis des composants de ceux-ci, soit avoir une fonction similaire à celle d'un de leurs constituants (par exemple un additif fonctionnel) et se substituer partiellement ou totalement à celui-ci.

Le radiotraceur doit avoir une radioactivité suffisante pour permettre des mesures précises et reproductibles. Le choix du radiotraceur est lié notamment à la quantité d'échantillon d'huile prélevée et à la sensibilité du détecteur utilisé. En d'autres termes, si le détecteur est peu sensible, la radioactivité de l'échantillon d'huile doit être élevée (radioactivité forte du radiotraceur ou concentration élevée d'un radiotraceur de radioactivité relativement faible). Par contre, si le détecteur utilisé a une sensibilté élevée, la radioactivité de l'échantillon d'huile peut être relativement plus faible.

Enfin, le radiotraceur doit être sélectionné de manière à ce que sa quantité en circulation dans le circuit d'huile du moteur soit, sur toute la durée du procédé, directement proportionnelle soit à la quantité d'huile de lubrification soit à la quantité de carburant en circulation dans le circuit d'huile du moteur.

Cette proportionnalité dépend des propriétés physico-chimiques du traceur radioactif et de celles du milieu liquide (huile lubrifiante ou carburant) dans lequel il est introduit initialement. En effet, pour refléter à tout moment la quantité d'huile lubrifiante ou la quantité de carburant dans l'échantillon d'huile (mélange huile/carburant), le traceur radioactif ne doit ni s'accumuler dans le mélange lorsque l'huile ou le carburant sont consommés, ni se consommer plus rapidement que ceux-ci, par exemple par évaporation, combustion ou décomposition thermique, ni être piégé en un endroit quelconque du moteur, tel que le filtre à huile.

A la lumière de ce qui précède, l'homme du métier choisira le radiotraceur tel que ses propriétés physico-chimiques (volatilité, stabilité thermique, réactivité chimique) soient en adéquation avec celles du milieu liquide dans lequel il est introduit et dont il doit refléter la quantité. L'homme du métier pourra notamment trouver un traceur approprié pour un milieu donné en soumettant un mélange traceur/huile lubrifiante ou un mélange traceur/carburant aux conditions de température et de pression qui règnent dans un moteur.

Comme expliqué ci-dessus, le procédé de la présente invention peut en principe être mis en oeuvre soit avec un carburant marqué par un radiotraceur, soit avec une huile de lubrification marquée.

L'utilisation d'un carburant radioactif implique le marquage d'un volume relativement important dudit carburant et la maîtrise du rejet, dans les gaz d'échappement, des produits de combustion du carburant incluant le radiotraceur.

Dans le cas de l'utilisation d'un lubrifiant radioactif, le volume est inférieur, les rejets éventuels dans les gaz d'échappement sont très limités et dépendent de la consommation d'huile.

Dans la suite de la description, le procédé de l'invention est expliqué plus en détail pour le mode de réalisation où l'huile de lubrification contient le traceur radioactif initialement introduit.

Les détecteurs utilisables sont des sondes de détection de rayonnements ionisants (rayons bêta, X ou gamma) pouvant être soit de type scintillateur liquide ou solide (cristal iodure de sodium NaI(T1), cristal BGO), soit de type semi-conducteur (cristal germanium, cristal CZT). On notera en outre que le détecteur peut déceler simultanément la présence de divers radiotraceurs. Lorsque la radioactivité de l'échantillon d'huile est élevée (radioactivité forte du radiotraceur ou une concentration élevée d'un radiotraceur de radioactivité faible), le détecteur ne nécessitera pas une sensibilité élevée. Par contre, lorsque la radioactivité de l'échantillon d'huile n'est pas élevée, le détecteur nécessitera une sensibilité plus élevée. Préférentiellement, et afin de limiter la quantité de radiotraceurs mis en oeuvre, on utilisera de préférence une sonde de mesure dont l'efficacité de détection est élevée, par exemple un cristal de type iodure de sodium de 3 x 3 pouces.

Ce type de détecteur peut exister sous forme compacte permettant la possibilité d'un dispositif embarqué sur véhicule.

En général, il est nécessaire d'amener un échantillon d'huile du circuit d'huile du moteur à tester vers une chambre de mesure de volume fixe qui se trouve dans le détecteur ou est située à proximité de celui-ci. Ce « prélèvement temporaire » suivi de la réintroduction de cet échantillon dans le circuit d'huile se fait de préférence par une dérivation. Pour des raisons pratiques relatives à la régulation du moteur, cette dérivation est de préférence située dans une zone du circuit d'huile qui est sous une pression d'huile faible, voire nulle.

Les signaux détectés par le détecteur sont ensuite traités par une série de moyens permettant de calculer le taux de dilution de l'huile lubrifiante par le carburant. Ces moyens comprennent notamment un moyen de traitement du signal détecté (par exemple un amplificateur, un filtre et un convertisseur analogique/numérique CAD), un moyen de traitement d'impulsions (par exemple un analyseur multi-canaux) et un moyen de stockage et de traitement des données acquises, par exemple un ordinateur PC. Pour le calcul du taux de dilution, le programme d'ordinateur devra bien entendu tenir compte de la diminution naturelle de la radioactivité du radiotraceur qui est directement liée au temps de demi-vie de celui-ci.

La dérivation de l'échantillon d'huile, la mesure de la radioactivité de l'échantillon dérivé et le traitement des résultats se font de préférence en continu, sur un moteur thermique en fonctionnement.

Le traceur radioactif utilisable dans la présente invention peut être soit un composé organique ou minéral d'un élément radioactif (radionucléide) soit l'élément radioactif lui-même qui est alors sous forme élémentaire. Toutefois, compte tenu des considérations ci-dessus concernant les propriétés physico-chimiques du traceur radioactif par rapport à celles de l'huile de lubrification, les formes moléculaires, organiques ou minérales, en particulier organiques, de radiotraceurs sont préférées par rapport aux formes élémentaires des radionucléides.

Le radiotraceur est donc choisi parmi les composés organiques ou minéraux ou les éléments remplissant les conditions indiquées précédemment (e.g.: caractère inerte vis-à-vis du lubrifiant ou substitution à l'un des composants du lubrifiant, radioactivité suffisante et proportionnalité huile/traceur). Toutefois, pour des raisons évidentes de manipulation et de protection de l'environnement, on choisira de préférence des traceurs contenant des radionucléides ayant une courte période, ou demi-vie, de préférence une période inférieure à 3 ans, en particulier inférieure à 1 an et encore plus préférentiellement inférieure à 30 jours. De cette manière, on évitera la production de déchets radioactifs à longue demi-vie.

Il est préférable que la période du radionucléide soit égale ou supérieure à la durée prévue de l'essai. L'ordinateur, grâce à la loi de décroissance radioactive pourra corriger facilement la valeur mesurée.

On peut citer à titre d'exemples de radionucléides ayant une période (indiquée entre parenthèses) appropriée : le ²²Na (2,61 ans), le ⁶⁵Zn (243,8 jours), le ⁴⁵Ca (165 jours), le ³⁵S (87,2 jours), le ³²P (14,3 jours), le ⁴⁷Ca (4,54 jours), le ⁹⁹Mo (65,9 heures), le ⁸²Br (35,3 heures), le ⁶⁴Cu (12,7 heures), le ^{99m}Tc (6,01 heures), le ²⁸Mg (20,91), le ⁶⁸Ge (270,95 jours), le ⁶⁹Ge (39h), le ⁷⁷Ge (11,30 heures), le ⁸⁵Sr (64,8 jours) et le ⁵⁶Co (77,3 jours).

Ces radiotraceurs sont en général produits artificiellement par réactions nucléaires, notamment par réaction d'activation. Cette activation se fait selon des méthodes familières à l'homme du métier, par exemple par exposition des éléments inactifs ou composés contenant lesdits éléments inactifs à une source de rayonnement neutronique, ou encore par exposition à un faisceau d'ions accélérés provenant d'un accélérateur de particules.

Selon les cas, les éléments inactifs ou composés contenant lesdits éléments inactifs sont activés soit avant leur incorporation dans l'huile de lubrification ou dans le carburant, soit au sein même de l'huile ou du carburant, c'est-à-dire en exposant l'huile ou le carburant contenant l'élément ou composé activable par exemple à un rayonnement neutronique ou à un faisceau de protons.

Une des options possibles pour obtenir des radionucléides artificiels est d'incorporer les éléments inactifs ou composés contenant lesdits éléments inactifs dans une quantité appropriée d'un vecteur (par exemple un solvant ou diluant tel qu'une huile), puis de soumettre ce mélange à l'activation et enfin de l'ajouter à l'huile lubrifiante ou au carburant.

Les radiotraceurs peuvent être des additifs utilisés habituellement dans les huiles de lubrification ou dans les carburants, tels que des agents anti-corrosion, des agents anti-oxydants, des agents modifiant la viscosité, des additifs lubrifiants, des colorants, des additifs abaissant le point d'écoulement, des additifs détergents ou dispersants. On peut citer à titre d'exemples de tels radiotraceurs fonctionnant comme un additif fonctionnel, le dithiophosphate de zinc, les sulfonates de calcium ou de magnésium, tels que les alkylsulfonates, arylsulfonates ou alkylarylsulfonates de calcium ou de magnésium, les phénates de calcium, les phénates de magnésium, les salicylates de calcium, les salicylates de magnésium.

Cependant, l'utilisation de radiotraceurs qui n'ont aucune fonction physique ou chimique dans le système de lubrification du moteur est toute aussi appropriée.

La Demanderesse a constaté que les traceurs radioactifs particulièrement intéressants pour une introduction dans l'huile de lubrification sont certains composés du germanium-69. Ces composés sont choisis par exemple parmi les tétra-alkylgermanes. Le point d'ébullition de ces tétra-alkylgermanes étant proportionnel à la longueur des chaînes alkyle, on utilisera avantageusement un mélange de tétraalkylgermanes ayant des chaînes alkyle telles que le point d'ébullition du mélange est situé dans l'intervalle de distillation de l'huile utilisée. A titre d'exemples, le tétrahexylgermane, le tétraheptylgermane et le tétraoctylgermane ont chacun un point d'ébullition comparable à celui d'un lubrifiant moteur classique.

### Exemple 1

### Suivi en continu du taux de dilution d'une huile lubrifiante par du gazole dans un circuit d'huile fermé

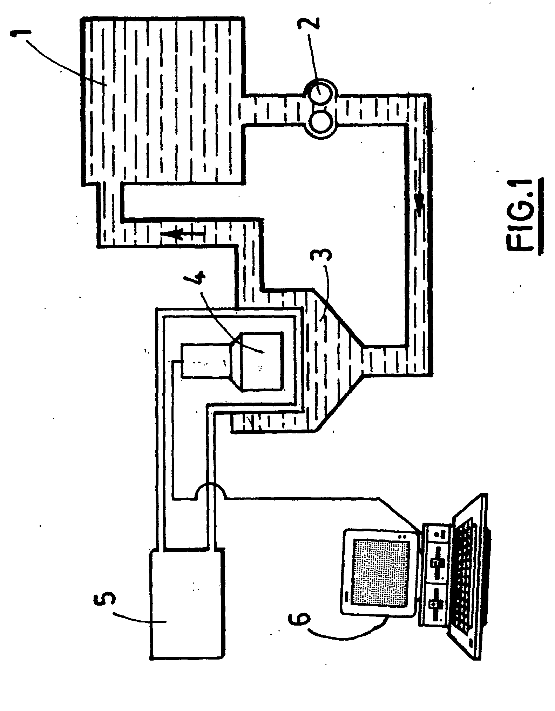

La figure 1 illustre le dispositif expérimental ayant servi à réaliser cet exemple. On fait circuler en circuit fermé, à l'aide d'une pompe (2) dont le débit est de 3 litres/min, un volume de 5,5 litres d'une huile lubrifiante de type minérale 15W40 (marque TOTAL). Celle-ci contient un traceur radioactif. Le traceur utilisé dans cet exemple est du ^{99m}Tc sous forme de pertechnétate de sodium Na^{99m}TcO₄ en solution aqueuse. Les caractéristiques de ce traceur radioactif sont les suivantes : émission gamma à 140 keV, intensité d'émission de 89 % et demi-vie de 6 heures. Afin de faciliter l'incorporation de la solution aqueuse dans l'huile lubrifiante, un dispersant d'eau commercialisé sous la marque « Bardahl Dispersant d'eau » est utilisé.

Le circuit fermé comprend, en série, un réservoir d'huile (1) ajustable en température simulant le carter du moteur à combustion interne et un cylindre à double paroi (3) d'une capacité de 1 litre constituant la chambre d'analyse, ce volume étant celui de l'échantillon d'huile prélevé. La température de l'huile est maintenue à 70°C. Au centre de la chambre d'analyse est placé un détecteur (4) standard à iodure de sodium NaI(T1) de 7,62 X 7,62 cm, soit 3 X 3 pouces, avec photomultiplicateur intégré, dont la température est stabilisée à 30°C par un groupe thermostatique (5). Le détecteur, qui est sensible au rayonnement gamma de 140 keV émis par le radiotraceur, est relié à un système d'acquisition et de traitement de données (6) constitué d'un préamplificateur de charge modèle 2007P de marque Canberra, d'un amplificateur de spectroscopie 2020 de marque Canberra, d'un convertisseur ADC modèle 8087 de marque Canberra et d'une carte multicanaux modèle S100 de marque Canberra également. Le logiciel mis en oeuvre dans cet exemple se nomme "IDSWear" et est commercialisé par la société Atlantic Nuclear Services (ANS) basée au Canada. Ce logiciel permet de suivre l'évolution temporelle du taux de comptage du détecteur dans une fenêtre d'énergie qui s'étend de 100 keV à 180 keV dans laquelle se situe le signal caractéristique du ^{99m}Tc.

En marquant les 5,5 litres d'huile de lubrification avec 1 MBq de ^{99m}Tc, le taux de comptage initial enregistré pour l'huile non-diluée est de 9020 coups/s, ce qui correspond à l'activité du litre d'huile contenu dans la chambre d'analyse. Après 50 minutes, 95 minutes et 120 minutes, on ajoute respectivement 55 ml de carburant, 200 ml de carburant et de nouveau 200 ml de carburant (gazole au standard européen) au niveau du réservoir d'huile, ce qui correspond à une dilution de 0,99 % vol, 4,4 % vol et 7,6 % vol.

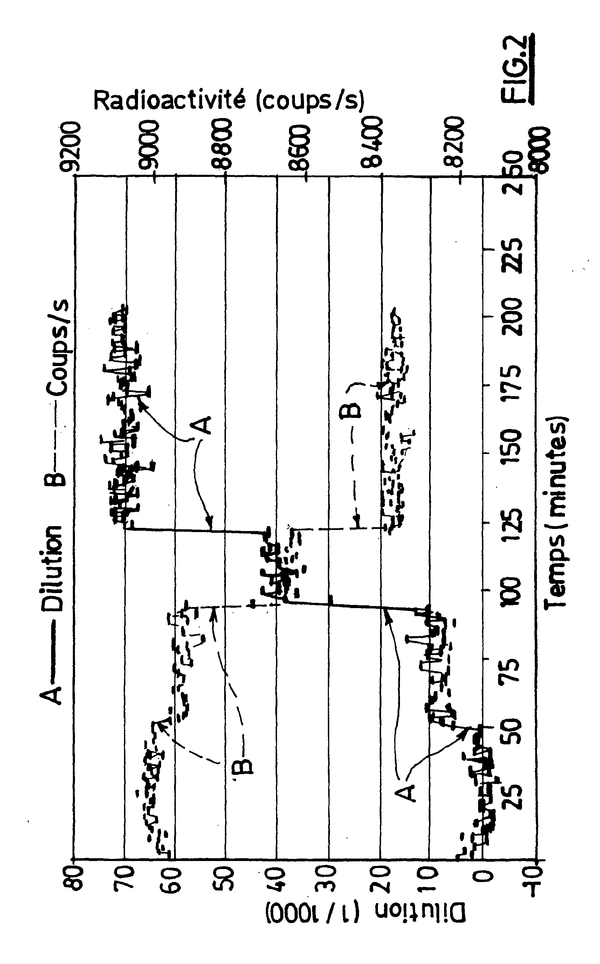

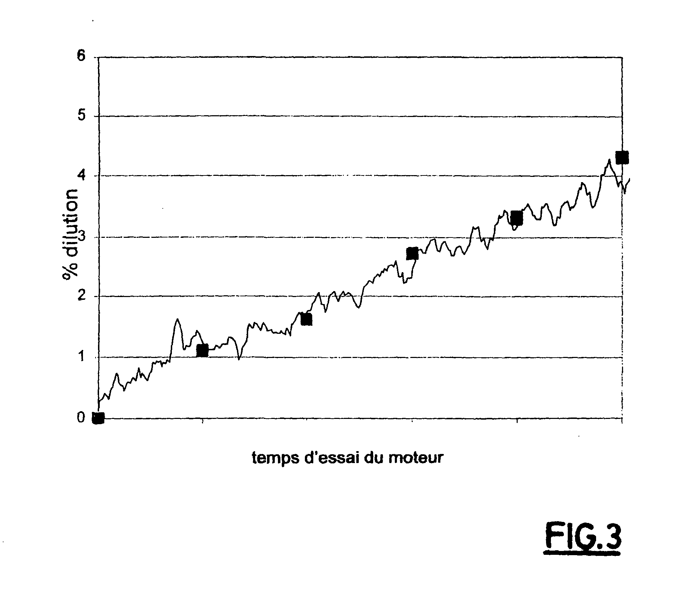

La figure 2 montre, à la fois, l'évolution en fonction du temps du taux de comptage au niveau de la chambre d'analyse (points gris clair) et de la dilution de l'huile lubrifiante par le gazole dans le mélange huile/gazole (points noirs). La courbe de dilution est calculée, par le logiciel, à partir des valeurs mesurées du taux de comptage, suivant une règle de proportionnalité : le taux de comptage initial correspond à une dilution de 0 % vol tandis qu'un taux de comptage nul correspond à une dilution de 100 % vol.

Cet exemple montre que le taux de comptage mesuré par le détecteur reflète correctement le taux de dilution imposé par addition de carburant (gazole), aux incertitudes de mesures près.

### Exemple 2

### Suivi en continu du taux de dilution d'une huile lubrifiante par du gazole dans un moteur diesel

Dans cet exemple, un banc d'essai moteur Diesel a été équipé d'un circuit de dérivation d'huile de faible débit. Celui-ci permet de prélever en continu du lubrifiant venant du carter d'huile vers le détecteur avant de le renvoyer vers le moteur. Le dispositif expérimental est quasi-identique à celui de l'exemple 1 à ceci près que le réservoir d'huile a été remplacé par un moteur Diesel. Pour cet essai, on fait fonctionner le moteur Diesel de façon usuelle à faible charge et faible régime afin favoriser la dilution par le gazole : 1500 tr/min, puissance 8,2 kW et température d'huile de 80°C.

Le traceur utilisé est cette fois du germanium 69 (Ge-69) sous la forme d'un composé organique soluble dans l'huile ayant une température d'ébullition qui se situe au milieu de l'intervalle de distillation de l'huile (à environ 450°C). Le traceur est utilisé à raison d'1 MBq dans 5 litres d'huile. Les caractéristiques de ce traceur radioactif sont les suivantes :
- émissions gamma : à 511 keV (47 %), 574 keV (13 %), 871 keV (12 %), 1106 keV (36 %) et 1336 keV (5 %)
- temps de demi-vie : 39 heures.

Le taux de comptage initial enregistré pour l'huile non-diluée est d'environ 950 coups/s. Ce taux de comptage s'accompagne d'une erreur statistique sur chaque point de mesure de l'ordre de 0,6 % .

Le moteur utilisé dans cet exemple est un moteur Diesel 4 cylindres à injection directe à rampe commune.

En parallèle à la mesure en continu, on effectue à intervalles réguliers des prélèvements d'un petit échantillon de lubrifiant et on détermine le taux de dilution de l'huile par le gazole par la méthode classique de chromatographie en phase gazeuse (type DIN 51380).

La courbe sur la figure 4 montre l'évolution du taux de dilution (exprimé en %) de l'huile par le gazole, mesuré en continu dans le carter d'huile par le système de détection par radio-traceurs (ligne continue) et mesuré à intervalles réguliers par chromatographie en phase gazeuse (carrés pleins). On peut constater que la mesure du taux de dilution en continu selon le procédé de l'invention est en adéquation parfaite avec la méthode de détermination discontinue selon l'état de la technique.

## Revendications

1. Procédé de détermination du taux de dilution de l'huile de lubrification d'un moteur à combustion interne par le carburant, **caractérisé par le fait que**
• l'on marque avec un traceur radioactif soit l'huile de lubrification soit le carburant,
• que l'on mesure à l'aide d'un détecteur sensible au rayonnement radioactif émis par le traceur radioactif, la radioactivité d'un échantillon d'huile, et
• que l'on transmet les résultats de ces mesures vers un ordinateur qui calcule à partir de ces résultats le taux de dilution de l'huile de lubrification par le carburant.

2. Procédé selon la revendication 1, **caractérisé par le fait que** c'est l'huile de lubrification qui contient le traceur radioactif.

3. Procédé selon la revendication 1, **caractérisé par le fait que** c'est le carburant qui contient le traceur radioactif.

4. Procédé selon l'une des revendications précédentes, **caractérisé par le fait que** l'échantillon d'huile dont on mesure la radioactivité est amené vers le détecteur puis réinjecté dans le circuit d'huile du moteur par une dérivation.

5. Procédé selon la revendication 4, **caractérisé par le fait que** la dérivation prélève l'échantillon d'huile au niveau d'une zone du circuit d'huile du moteur qui n'est pas sous pression ou sous une faible pression d'huile.

6. Procédé selon l'une quelconque des revendications précédentes, **caractérisé par le fait que** le traceur radioactif est un composé organique ou minéral d'un élément radioactif, de préférence un composé organique d'un élément radioactif.

7. Procédé selon l'une des revendications précédentes, **caractérisé par le fait que** l'élément radioactif a une période, ou demi-vie, inférieure à 3 ans, de préférence inférieure à 1 an, et en particulier inférieure à 30 jours.

8. Procédé selon la revendication 7, **caractérisé par le fait que** l'élément radioactif est choisi parmi le ²²Na, le ⁶⁵Zn, le ⁴⁵Ca, le ³⁵S, le ³²P, le ⁴⁷Ca, le ⁹⁹Mo, le ⁸²Br, le ⁶⁴Cu, le ^{99m}Tc, le ²⁸Mg, le ⁶⁸Ge, le ⁶⁹Ge, le ⁷⁷Ge, le ⁸⁵Sr et le ⁵⁶Co.

9. Procédé selon la revendication 8, **caractérisé par le fait que** le radiotraceur est choisi parmi les tétra-alkylgermanes contenant du ⁶⁹Ge, de préférence parmi le tétrahexylgermane, le tétraheptylgermane et le tétraoctylgermane, ou un mélange de ceux-ci.

10. Procédé selon l'une quelconque des revendications précédentes, **caractérisé par le fait que** le détecteur est une sonde de détection de rayonnements ionisants.

11. Dispositif de suivi du taux de dilution de l'huile de lubrification d'un moteur à combustions interne par le carburant, **caractérisé par le fait qu'**il comprend
• un moteur à combustion interne, lubrifié par une huile de lubrification et alimenté par un mélange air/carburant, soit l'huile de lubrification soit le carburant étant marqués par un traceur radioactif,
• un moyen permettant le prélèvement temporaire et la réinjection, en continu ou discontinu, d'un échantillon d'huile du circuit d'huile du moteur,
• à proximité immédiate de ce moyen de prélèvement temporaire et de réinjection, un détecteur sensible au rayonnement radioactif émis par le traceur radioactif présent dans l'échantillon d'huile, et
• relié audit détecteur, un ordinateur programmé pour calculer, à partir des résultats des mesures de radioactivité de l'échantillon d'huile, fournis par ledit détecteur, le taux de dilution de l'huile de lubrification par le carburant.

12. Dispositif selon la revendication 11, **caractérisé par le fait que** c'est l'huile de lubrification qui contient le traceur radioactif.

13. Dispositif selon la revendication 11, **caractérisé par le fait que** c'est le carburant qui contient le traceur radioactif.

14. Dispositif selon l'une des revendications 11 et 12, **caractérisé par le fait que** le moyen permettant le prélèvement temporaire et la réinjection, en continu ou discontinu, d'un échantillon d'huile est une dérivation.

15. Dispositif selon la revendication 14, **caractérisé par le fait que** la dérivation prélève et réinjecte l'échantillon d'huile au niveau d'une zone du circuit d'huile du moteur qui n'est pas sous pression ou sous une faible pression d'huile.

16. Dispositif selon l'une quelconque des revendications 11 à 15, **caractérisé par le fait que** le traceur radioactif est un composé organique ou minéral d'un élément radioactif, de préférence un composé organique d'un élément radioactif.

17. Dispositif selon l'une des revendications 11 à 16, **caractérisé par le fait que** l'élément radioactif a une période, ou demi-vie, inférieure à 3 ans, de préférence inférieure à 1 an, et en particulier inférieure à 30 jours.

18. Dispositif selon la revendication 17, **caractérisé par le fait que** l'élément radioactif est choisi parmi le ²²Na, le ⁶⁵Zn, le ⁴⁵Ca, le ³⁵S, le ³²P, le ⁴⁷Ca, le ⁹⁹Mo, le ⁸²Br, le ⁶⁴Cu, le ^{99m}Tc, le ²⁸Mg, le ⁶⁸Ge, le ⁶⁹Ge, le ⁷⁷Ge, le ⁸⁵Sr et le ⁵⁶Co.

19. Dispositif selon la revendication 18, **caractérisé par le fait que** le radiotraceur est choisi parmi les tétra-alkylgermanes contenant du ⁶⁹Ge, de préférence parmi le tétrahexylgermane, le tétraheptylgermane et le tétraoctylgermane, ou un mélange de ceux-ci.

20. Dispositif selon l'une quelconque des revendications 11 à 19, **caractérisé par le fait que** le détecteur est une sonde de détection de rayonnements ionisants.

## Claims

1. Method for determining the rate of dilution of the lubricating oil of an internal combustion engine by the fuel, **characterized in that**
• either the lubricating oil or the fuel is labelled with a radioactive tracer,
• the radioactivity of an oil sample is measured using a detector sensitive to the radioactive radiation emitted by the radioactive tracer, and
• the results of these measurements are transmitted to a computer, which calculates the rate of dilution of the lubricating oil by the fuel from these results.

2. Method according to Claim 1, **characterized in that** it is the lubricating oil which contains the radioactive tracer.

3. Method according to Claim 1, **characterized in that** it is the fuel which contains the radioactive tracer.

4. Method according to one of the preceding claims, **characterized in that** the oil sample of which the radioactivity is measured is sent to the detector and then reinjected into the engine oil circuit via a bypass.

5. Method according to Claim 4, **characterized in that** the bypass takes the oil sample from a zone of the engine oil circuit which is not under pressure or is under a low oil pressure.

6. Method according to any one of the preceding claims, **characterized in that** the radioactive tracer is an organic or inorganic compound of a radioelement, preferably an organic compound of a radioelement.

7. Method according to one of the preceding claims, **characterized in that** the radioelement has a half-life shorter than 3 years, preferably shorter than 1 year, and in particular shorter than 30 days.

8. Method according to Claim 7, **characterized in that** the radioelement is selected from ²²Na, ⁶⁵Zn, ⁴⁵Ca, ³⁵S, ³²P, ⁴⁷Ca, ⁹⁹Mo, ⁸²Br, ⁶⁴Cu, ^{99m}Tc, ²⁸Mg, ⁶⁸Ge, ⁶⁹Ge, ⁷⁷Ge, ⁸⁵Sr and ⁵⁶Co.

9. Method according to Claim 8, **characterized in that** the radioactive tracer is selected from tetra-alkylgermanes containing ⁶⁹Ge, preferably from tetrahexylgermane, tetraheptylgermane, tetraoctylgermane, or a mixture thereof.

10. Method according to any one of the preceding claims, **characterized in that** the detector is a probe for detecting ionizing radiation.

11. Device for monitoring the rate of dilution of the lubricating oil of an internal combustion engine by the fuel, **characterized in that** it comprises:
• an internal combustion engine, lubricated by a lubricating oil and supplied with an air/fuel mixture, either the lubricating oil or the fuel being labelled by a radioactive tracer,
• means for temporarily sampling and reinjecting an oil sample from the engine oil circuit, continuously or intermittently,
• immediately next to the said temporary sampling and reinjection means, a detector sensitive to the radioactive radiation emitted by the radioactive tracer present in the oil sample, and
• connected to the said detector, a computer programmed to calculate the rate of dilution of the lubricating oil by the fuel, from the results of the radioactive measurements of the oil sample, supplied by the said detector.

12. Device according to Claim 11, **characterized in that** it is the lubricating oil which contains the radioactive tracer.

13. Device according to Claim 11, **characterized in that** it is the fuel which contains the radioactive tracer.

14. Device according to either of Claims 11 and 12, **characterized in that** the means for temporarily sampling and reinjecting an oil sample, continuously or intermittently, is a bypass.

15. Device according to Claim 14, **characterized in that** the bypass takes and reinjects the oil sample from a zone of the engine oil circuit which is not under pressure or is under a low oil pressure.

16. Device according to any one of Claims 11 to 15, **characterized in that** the radioactive tracer is an organic or inorganic compound of a radioelement, preferably an organic compound of a radioelement.

17. Device according to one of Claims 11 to 16, **characterized in that** the radioelement has a half-life shorter than 3 years, preferably shorter than 1 year, and in particular shorter than 30 days.

18. Device according to Claim 17, **characterized in that** the radioelement is selected from ²²Na, ⁶⁵Zn, ⁴⁵Ca, ³⁵S, ³²P, ⁴⁷Ca, ⁹⁹Mo, ⁸²Br, ⁶⁴Cu, ^{99m}Tc, ²¹Mg, ⁶⁸Ge, ⁶⁹Ge, ⁷⁷Ge, ⁸⁵Sr and ⁵⁶Co.

19. Device according to Claim 18, **characterized in that** the radioactive tracer is selected from tetra-alkylgermanes containing ⁶⁹Ge, preferably from tetrahexylgermane, tetraheptylgermane, tetraoctylgermane, or a mixture thereof.

20. Device according to any one of Claims 11 to 19, **characterized in that** the detector is a probe for detecting ionizing radiation.

## Patentansprüche

1. Verfahren zum Bestimmen des Verdünnungsgrads von Schmieröl eines Verbrennungsmotors durch den Brennstoff, **dadurch gekennzeichnet, dass**
das Schmieröl oder der Brennstoff mit einem Radioindikator markiert wird,
mittels eines Detektors, der für die von dem Radioindikator abgegebene radioaktive Strahlung empfindlich ist, die Radioaktivität eine Probe des Öls gemessen wird und
die Ergebnisse dieser Messungen zu einem Rechner übertragen werden, der ausgehend von diesen Ergebnissen den Verdünnungsgrad des Schmieröls durch den Brennstoff berechnet.

2. Verfahren gemäß Anspruch 1, **dadurch gekennzeichnet, dass** das Schmieröl den Radioindikator enthält.

3. Verfahren gemäß Anspruch 1, **dadurch gekennzeichnet, dass** der Brennstoff den Radioindikator enthält.

4. Verfahren gemäß einem der vorigen Ansprüche, **dadurch gekennzeichnet, dass** die Ölprobe, deren Radioaktivität gemessen wird, durch eine Abzweigung zu dem Detektor gebracht wird und dann in den Ölkreislauf des Motors zurückgebracht wird.

5. Verfahren gemäß Anspruch 4, **dadurch gekennzeichnet, dass** die Abzweigung die Ölprobe auf dem Niveau einer Zone des Ölkreislaufs des Motors entnimmt, die nicht unter Druck oder unter einem schwachen Öldruck steht.

6. Verfahren gemäß einem der vorigen Ansprüche, **dadurch gekennzeichnet, dass** der Radioindikator eine organische oder mineralische Verbindung eines radioaktiven Elements ist und vorzugsweise eine organische Verbindung des radioaktiven Elements.

7. Verfahren gemäß einem der vorigen Ansprüche, **dadurch gekennzeichnet, dass** das radioaktive Element eine Halbwertszeit von weniger als 3 Jahren aufweist, vorzugsweise von weniger als 1 Jahr und in weiter bevorzugter Weise von weniger als 30 Tagen.

8. Verfahren gemäß Anspruch 7, **dadurch gekennzeichnet, dass** das radioaktive Element gewählt ist aus ²²Na, ⁶⁵Zn, ⁴⁵Ca, ³⁵S, ³²P, ⁴⁷Ca, ⁹⁹Mo, ⁸²Br, ⁶⁴Cu, ^{99m}Tc, ²⁸Mg, ⁶⁸Ge, ⁶⁹Ge, ⁷⁷Ge, ⁸⁵Sr et ⁵⁶Co.

9. Verfahren gemäß Anspruch 8, **dadurch gekennzeichnet, dass** der Radioindikator gewählt ist aus Tetraalkylgermanen, die ⁶⁹Ge enthalten, vorzugsweise aus Tetrahexylgermanen, Tetraheptylgermanen und Tetraoctylgermanen oder einer Mischung aus diesen.

10. Verfahren gemäß einem der vorigen Ansprüche, **dadurch gekennzeichnet, dass** der Detektor eine Sonde zum Detektieren ionisierender Strahlung ist.

11. Vorrichtung zum Überwachen des Verdünnungsgrads von Schmieröl eines Verbrennungsmotors durch den Brennstoff, **dadurch gekennzeichnet, dass** sie enthält:
einen Verbrennungsmotor, der durch ein Schmieröl geschmiert ist und durch eine Mischung von Brennstoff und Luft gespeist ist, wobei das Schmieröl oder der Brennstoff mit einem Radioindikator markiert sind,
ein Mittel, das die temporäre Entnahme und Wiedereinführung einer Ölprobe des Ölkreislaufs des Motors auf kontinuierliche oder diskontinuierliche Weise ermöglicht,
in unmittelbarer Nähe zu dem Mittel zur temporären Entnahme und Wiedereinführung einen Detektor, der für die von dem in der Ölprobe befindlichen Radioindikator abgegebene radioaktive Strahlung empfindlich ist, und
verbunden mit dem Detektor einen Rechner, der programmiert ist zum Berechnen des Verdünnungsgrads des Schmieröls durch den Brennstoff ausgehend von den Ergebnissen der Messung der Radioaktivität der Ölprobe, die von dem Detektor geliefert werden.

12. Vorrichtung gemäß Anspruch 11, **dadurch gekennzeichnet, dass** das Schmieröl den Radioindikator enthält.

13. Vorrichtung gemäß Anspruch 11, **dadurch gekennzeichnet, dass** der Brennstoff den Radioindikator enthält.

14. Vorrichtung gemäß Anspruch 11 oder 12, **dadurch gekennzeichnet, dass** das Mittel, das die temporäre Entnahme und Wiedereinführung einer Ölprobe auf kontinuierliche oder diskontinuierliche Weise ermöglicht, eine Abzweigung ist.

15. Vorrichtung gemäß Anspruch 14, **dadurch gekennzeichnet, dass** die Abzweigung die Ölprobe auf dem Niveau einer Zone des Ölkreislaufs des Motors entnimmt, die nicht unter Druck oder unter einem schwachen Öldruck steht.

16. Vorrichtung gemäß einem der Ansprüche 11 bis 15, **dadurch gekennzeichnet, dass** der Radioindikator eine organische oder mineralische Verbindung eines radioaktiven Elements ist und vorzugsweise eine organische Verbindung des radioaktiven Elements.

17. Vorrichtung gemäß einem der Ansprüche 11 bis 16, **dadurch gekennzeichnet, dass** das radioaktive Element eine Halbwertszeit von weniger als 3 Jahren aufweist, vorzugsweise von weniger als 1 Jahr und in weiter bevorzugter Weise von weniger als 30 Tagen.

18. Vorrichtung gemäß Anspruch 17, **dadurch gekennzeichnet, dass** das radioaktive Element gewählt ist aus ²²Na, ⁶⁵Zn, ⁴⁵Ca, ³⁵S, ³²P, ⁴⁷Ca, ⁹⁹Mo, ⁸²Br, ⁶⁴Cu, ^{99m}Tc, ²⁸Mg, ⁶⁸Ge, ⁶⁹Ge, ⁷⁷Ge, ⁸⁵Sr et ⁵⁶Co.

19. Vorrichtung gemäß Anspruch 18, **dadurch gekennzeichnet, dass** der Radioindikator gewählt ist aus Tetraalkylgermanen, die ⁶⁹Ge enthalten, vorzugsweise aus Tetrahexylgermanen, Tetraheptylgermanen und Tetraoctylgermanen oder einer Mischung aus diesen.

20. Vorrichtung gemäß einem der Ansprüche 11 bis 19, **dadurch gekennzeichnet, dass** der Detektor eine Sonde zum Detektieren ionisierender Strahlung ist.
